# EUROPEAN PATENT APPLICATION

(11) **EP 3 660 144 A1**
(43) Date of publication of application: **03.06.2020**
(21) Application number: 18306591.1
(22) Date of filing: 30.11.2018
(51) Int. Cl.: C12N 5/077, C12N 5/00

(54) **OSTEOBLASTS DERIVED FROM ORAL NEUROECTODERMAL STEM CELLS AND THEIR USE IN JAW REPAIR**

(71) Applicant: ASSISTANCE PUBLIQUE, HOPITAUX DE PARIS, 75004 Paris (FR); Université de Paris 5 René Descartes, 75006 Paris (FR); Université Paris Diderot Paris 7, 75013 Paris (FR); Université Paris-Est Créteil Val de Marne, 94010 Creteil Cedex (FR)
(72) Inventor: FOURNIER, Benjamin, 75012 Paris (FR); FERRE, François, 75011 Paris (FR); GOGLY, Burno, 77510 Hondevilliers (FR); NASSIF, Ali, 78500 Sartrouville (FR); TAÏHI, Ihsène, 78500 Sartrouville (FR)
(74) Representative: Novagraaf Technologies

(57) **Abstract**

The present invention concerns a method for inducing differentiation of neuroectodermal oral stem cells, in particular from gingival tissue (GSCs), into osteoblasts by culturing them in an optimal serum-free medium supplemented by necessary components such as platelet lysate, growth hormone, heparin, and/or growth factors. The invention method provides osteoblasts for cell therapy, particularly for the restoration of bone defects in maxillary bones.

## Description

### Technical field of the invention

The present invention concerns a method for inducing differentiation of neuroectodermal oral stem cells, in particular derived from gingival tissue (GSCs), into osteoblasts by culturing them in an serum-free optimal medium supplemented by necessary components such as platelet lysate, growth hormone, heparin, and/or growth factors. The method of the present invention provides osteoblasts for cell therapy, particularly for the restoration of bone defects in maxillary bones.

In the description below, the references in square brackets ([ ]) refer to the list of references at the end of the text.

### State of the art

Dentists and maxillofacial surgeons are daily confronted with the problems of bone loss in the jaws. These bone defects range from a few millimetres to several centimetres, ranging from small periodontal defects, post-extraction leakages to defects caused by congenital malformations, tumour resections or trauma. For small maxillary bone defects (<1cm), WHO has established that the global economic cost incurred as a result was estimated at $442 billion in 2010, with carious and periodontal diseases as the main causes (Listl et al., 2015)[1]. WHO estimates that the average rates of total edentulism at 60 years of age are 16-67% in Europe, 26% in the United States and 58% in Canada (Petersen and Yamamoto, 2005)[2]. Other more mutilating malformative and tumour pathologies combine with other causes of jawbone defects, and the main treatment remains surgical treatment (Gundlach and Christina, 2006; Montero and Patel, 2015)[3, 4]. This treatment is mainly based on bone grafts, often autogenous, and requiring an extra-oral donor site for massive bone loss. These autografts are therefore often very mutilating due to the presence of two intervention sites. In addition, their bone regeneration capacities remain useful and effective but not optimal in ectopic grafts, given the difference in expression of homeogens between maxillary and appendicular bones (Leucht et al, 2008)[5], which also differ in their phenotypes (Jacques et al., 2014)[6] as well as in their embryological origins: jaw bones derive from neural crests (NC), while appendicular bones derive from mesoderm (Nassif, 2016)[7].

To overcome this situation, cell therapy appears to be an attractive alternative, in line with the evolution of the treatment of major bone defects in surgical orthopaedics. It is based on fundamental knowledge and practical use of adult stem cells. Mesenchymal stem cell (MSC) tissue engineering could restore voluminous substance losses from different tissues in humans, including maxillary bones. These MSCs, whatever their isolation sites and origins (bone marrow, adipose tissue, etc...) have significant plasticity and capacities for self-renewal and cell differentiation. They also regulate inflammatory reactions, by acting on different factors (regulatory T lymphocytes, NK, macrophages, etc...) through various mechanisms, giving hope for many therapeutic purposes (Najar et al., 2016)[8]. In the field of bone regeneration, many stem cell reservoirs have historically been exploited, particularly bone marrow. On the orthopaedic scene, other sources are currently being explored, including adipose tissue.

Historically, MSCs have been considered equivalent throughout the skeleton and clinically exploited without considering their anatomical origin. However, bone cell therapy based on MSCs from bone marrow is not the appropriate therapy given the differences in embryonic origin, phenotype and expression of the homeogens that control bone healing. Indeed, the axial and appendicular bones derive from the mesoderm and the maxillofacial bone from the neuroectoderm. In addition, in the adult skeleton, Hox genes are expressed in MSCs, and genetic analyses have provided evidence that these genes work during the bone fracture healing process, which could affect bone regeneration outcomes by transplanting an MSC type from a region with a different Hox profile (Seifert et al., 2015)[9].

Thus, cell therapy using neuroectodermal stem cells (i.e. derived from CNs) could offer a better alternative to cells from mesodermal sources (bone marrow) and autologous transplantation. However, the current pitfall is to obtain a source of MSC in sites close to the jaw bones. Clinical work has already established the bone regeneration potential of these cells (d'Aquino et al., 2009) [10] using dental pulp stem cells (DPSC). This source involves the prior extraction of a tooth and seems difficult to apply in daily practice. However, it remains interesting in children with temporary teeth or in patients with non-functional wisdom teeth.

Inventors have recently isolated oral neuroectodermal stem cells from gingival tissue (GSC) (Fournier et al., 2010) [11]. These GSCs have the same embryological origin as the jaw bones, i.e. the neural crests (NCs). Their ability to differentiate, particularly in osteoblasts, and the limiting and non-selective access for the patient during routine surgical procedures under local anaesthesia seem to offer a more realistic and practical alternative. Indeed, gum healing is considered embryo-like. Cells are easy to grow and stem cells are particularly resistant. Gingival tissue is probably the most aggressive tissue of oragnism (mechanical, thermal, chemical and bacterial aggressions), however no scars are visible in the oral cavity even after dental extractions. Among other things, these cells can modulate the inflammatory response by increasing regulatory T cells, thus making it possible to treat animal models of rheumatoid arthritis and intestinal colitis (Zhang et al., 2009; Chen et al., 2013) [12, 13]. The immunomodulatory property of the GSCs would finally make it possible to perpetuate cell transplant by controlling the inflammatory phenomena associated with this transplant. This could also be used for various therapeutic purposes (autoimmune diseases, tissue regeneration other than bone).

The use of GSCs in bone cell therapy to restore maxillary bone defects therefore seems to be a *priori* a very good perspective that amply meets the specifications necessary for their use. Multiple studies have described the potential for bone reconstruction of GSCs (Tomar et al., 2010; Wang et al., 2011) [14, 15]. However, many adjustments are nevertheless necessary, and suitable cultivation methods have yet to be defined before they can be transferred to humans.

### Description of the invention

As part of the regeneration of bone defects in maxillary bones in humans, the Inventors have therefore developed a GSC culture protocol without the use of fetal calf serum (FCS): the most widely used supplement in conventional MSC cell culture media, rich in proteins and growth factors necessary for cell proliferation and differentiation, but with a risk of transmission of xenogenic immunogenic factors or emerging zoonotic diseases when used in humans. While the purpose is to be able to use GSCs in human bone cell therapy.

To do this, the Inventors used a human substitute for FCS, represented by platelet lysate (PL) (Naveau et al., 2011) [16], by combining it with a cocktail of growth factors, and selected an ideal medium for serum-free GSC isolation and cell proliferation (phase 1) at rates equivalent to those obtained with FCS. Osteoblastic differentiation (phase 2) and preservation of the immunomodulatory properties of GSCs have also been studied under these new conditions.

The ultimate clinical objective is to isolate GSCs from an individual, amplify them *in vitro,* differentiate them towards the osteoblastic pathway, using the protocol of the invention, then re-implant them in the site of interest (e.g. maxillary bone defect) of the same individual, in order to reconstruct the bone site in an optimal and sustainable manner.

An object of the present invention is therefore a method for inducing differentiation of oral neuroectodemal stem cells into osteoblasts, said method comprising or consisting of:
(a) a step of cell proliferation of neuroectodermal oral stem cells in a serum-free base culture medium supplemented with platelet lysate (PL) and human growth hormone (GH);
b) an osteoblastic differentiation step of the cell culture resulting from step a) in a serum-free base culture medium supplemented with a platelet lysate (PL) and at least one osteoblastic differentiation factor.

According to a particular embodiment of the method of the present invention, PL is used at a concentration ranging from 2-20%, preferably at a concentration of 10%.

According to a particular embodiment of the method of the present invention, the culture of step a) is carried out until a confluence of 80% is obtained.

According to a particular embodiment of the method of the present invention, the culture of step b) is carried out for 21 to 28 days.

According to a particular embodiment of the method of the present invention, the culture medium of steps a) and b) is further supplemented with heparin. Preferably, the culture medium of step a) contains a concentration ranging from 1-2 IU/ml heparin, preferably of 2 IU/ml heparin and/or the culture medium of step b) contains a concentration ranging from 0.1-0.6 IU/ml heparin, preferably of 0.6 IU/ml.

According to a particular embodiment of the method of the present invention, the said at least one osteoblastic differentiation factor is chosen from the group consisting of corticosteroids and glycerol phosphoric esters. Preferably, said at least one osteoblastic differentiation factor is dexamethasone and/or β-glycerophosphate.

According to a particular embodiment of the method of the present invention, neuroectodemic oral stem cells are derived from gingival tissue.

Another object of the present invention is also a cellular population comprising or consisting of osteoblasts produced by the method according to the invention, said osteoblasts expressing the Msx2 homeogen specific for remodeling the craniofacial area (which is not the case for other osteoblasts obtained from other stem cells and/or other art processes). Moreover, for appendicular bones, it should be noted that Msx2 is not expressed.

Another object of the present invention is also the cell population according to the invention, for use in cell therapy, preferably for the restoration of bone defects in the maxillary bones.

Another object of the present invention is also a culture medium for the proliferation of oral neuroectodermal stem cells comprising or consisting of a serum-free base culture medium supplemented with platelet lysate (PL) and human growth hormone (GH), and optionally heparin, for example at a concentration of 1-2 IU/ml, preferably at a concentration of 2 IU/ml.

Another object of the present invention is also a culture medium for the differentiation of oral neuroectodermal stem cells into osteoblasts comprising or consisting of a serum-free base culture medium supplemented with a platelet lysate (PL) and at least one osteoblastic differentiation factor, and optionally heparin, for example at a concentration of 0.1-0.6 IU/ml, preferably at a concentration of 0.6 IU/ml.

According to a particular method of the present invention, the said at least one osteoblastic differentiation factor of the culture medium for the differentiation of neuroectodermal oral stem cells into osteoblasts is chosen from the group consisting of corticosteroids and glycerol phosphoric esters. Preferably said at least one osteoblastic differentiation factor is dexamethasone and/or β-glycerophosphate.

### Brief description of the figures

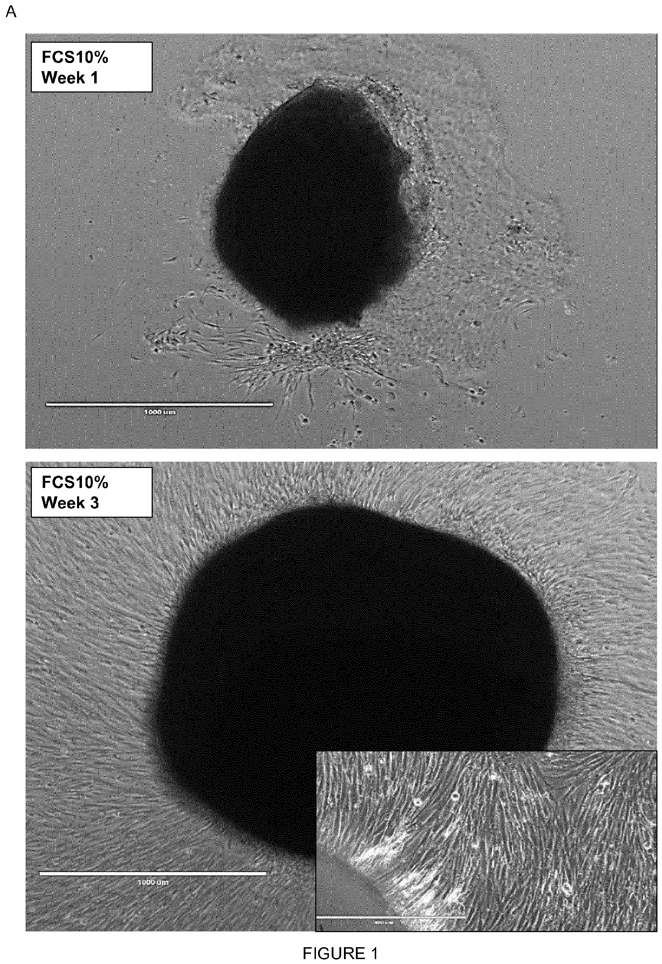

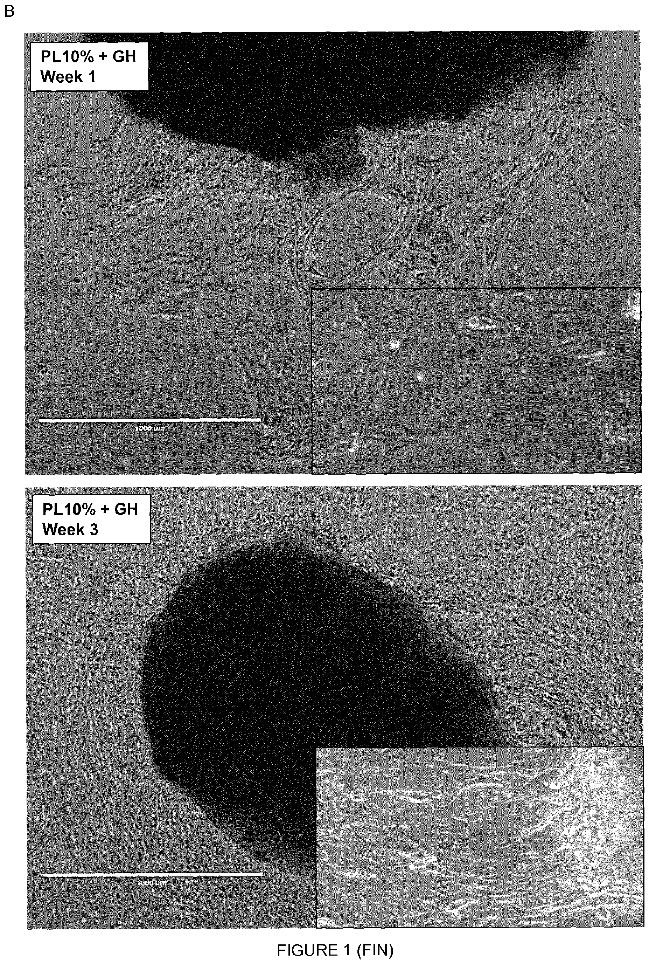

- Figure 1 shows optical microscopy photos (no staining) representing the isolation of (A) GSCs in the base medium with FCS 10% at week 1 and week 3, (B) GSCs in the new serum-free medium supplemented with PL10%+GH at week 1 and week 3; by the explant method well-known to the skilled person.

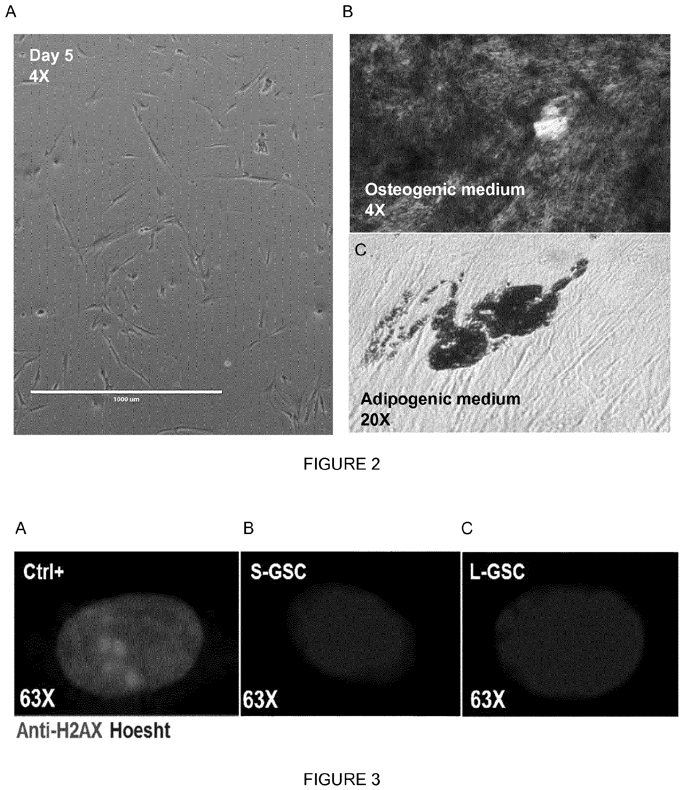

- Figure 2 shows optical microscopy photos representing the possible freeze/thaw of GSCs in the PL on day 5 of re-cultivation after thawing (A) no staining, 4x magnification (B) Alizarin Red S staining (specific of osteoblasts) according to a protocol well-known to the skilled person, 4x magnification (C) Sudan black B staining (specific of adipocytes) according to a protocol well-known to the skilled person, 20x magnification.
- Figure 3 shows optical microscopy photos (under anti-H2AX-Hoesht staining according to a protocol well-known to the skilled person, 63x magnification) comparing the possible occurrence of significant DNA damage in (A) UV-irradiated gingival cells (Ctrl+) (B) S-GSCs cells, and (C) L-GSCs cells.

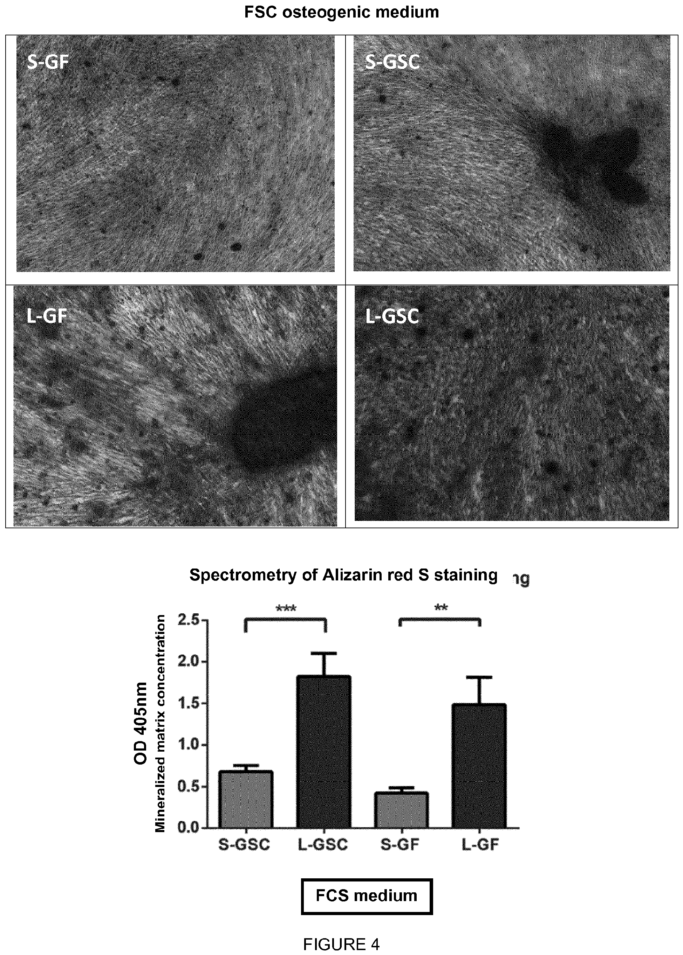

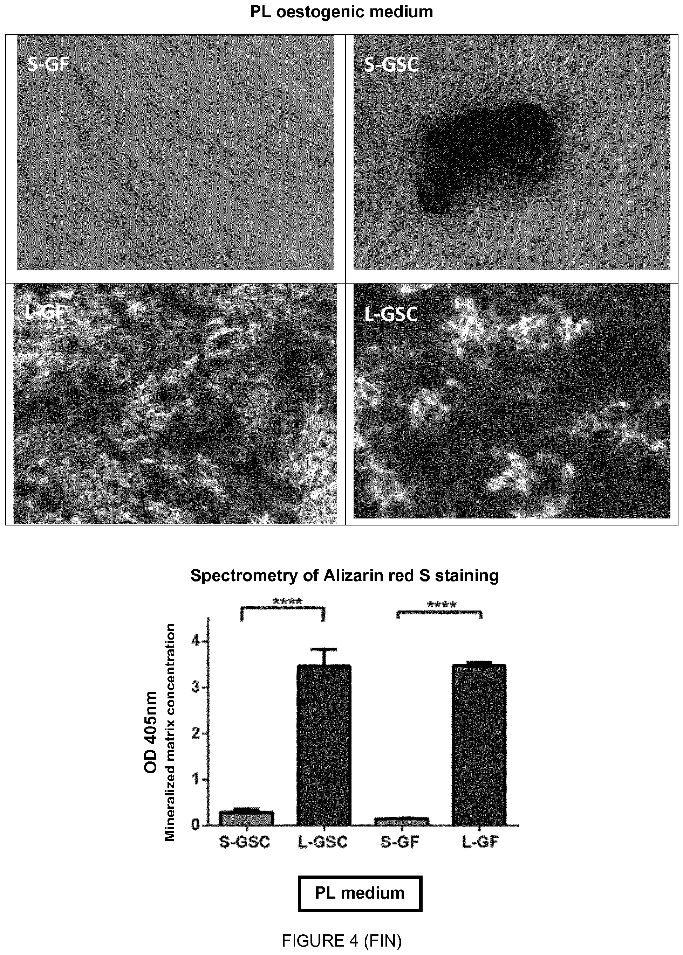

- Figure 4 shows optical microscopy photos (Alizarin red S staining according to a protocol well-known to the skilled person) representing the osteoblastic differentiation (phase 2) of gingival fibroblasts (S-GF or L-GF depending on whether they come from a proliferation medium with FCS or PL medium, respectively) and gingival stem cells (S-GSCs or L-GSCs depending on whether they come from a proliferation medium with FCS or PL medium, respectively) in 2D, in an inductive medium chosen from FSC osteogenic medium or serum-free PL osteogenic medium, over 28 days.

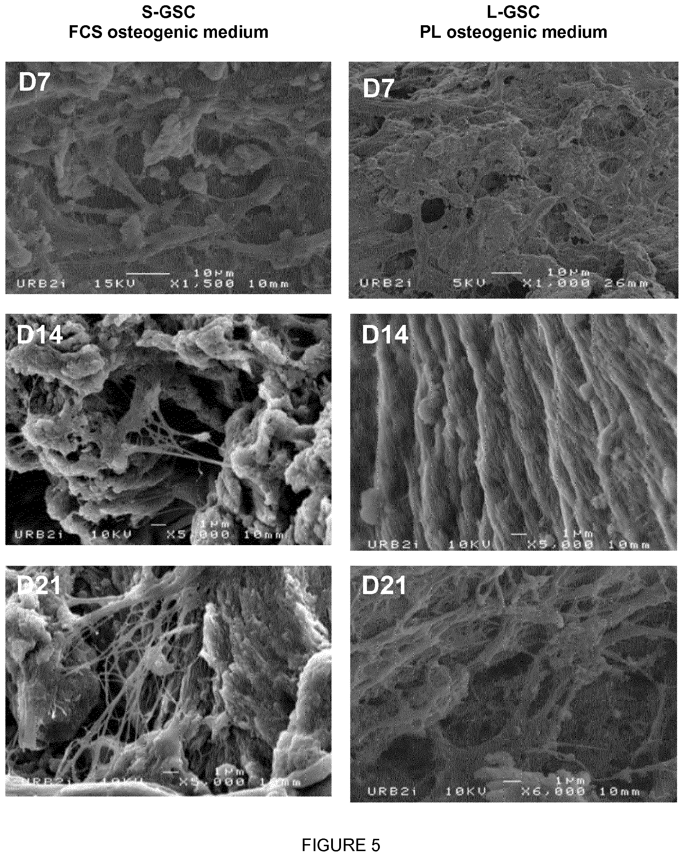

- Figure 5 shows electronic microscopy photos (no staining) representing the osteoblastic differentiation (phase 2) of L-GSCs or S-GSCs depending on whether they come from a proliferation medium with FCS or PL medium, respectively, in an inductive medium chosen from FSC osteogenic medium or serum-free PL osteogenic medium, in 3D, over 7, 14 and 21 days, with human allogeneic bone particles according to a protocol well-known to the skilled person.

### EXAMPLES

### EXAMPLE 1 : PROLIFERATION AND CELLULAR DIFFERENTIATION OF GSC IN SERUM-FREE MEDIUM

### I- Protocol and summary of results

| | **Culture type** | **basic medium** | **PL level** | **Cell culture supplements** | **Change of culture medium** | **Culture time** |
|---|---|---|---|---|---|---|
| **Cell proliferation in serum-free medium (PL + GH medium)** | 2D | DMEM-LG GlutaMAX® and pyruvate supplement (Gibco-Life Technologies, Carlsbad, CA, USA) | 10% | 4ng/ml GH (Peprotech, USA) | 2x per week | up to a confluence of 80% |
| | | | | L-Ascorbic acid 2-phosphate (50µg/ml; Sigma-Aldrich) | | |
| | | | | 10ml/l Penicillin-Streptomycin (5UI/ml, Gibco) | | |
| | | | | 1% non-essential aminoacids (NEAA; Gibco) | | |
| | | | | 2,5mg/l d'amphotericin B (250µg/ml ; Gibco) | | |
| | | | | Heparin 2UI/ml | | |
| *Proliferation test* | The PL + GH medium has proliferation rates equivalent to or higher than the conventional medium supplemented with 10% FCS | | | | | |
| *Primary culture* | Isolation of GSCs in primary culture is possible without the use of FCS with conservation of their properties (called L-GSC in contrast to S-GSCs isolated in the FCS) | | | | | |
| *CFU-F* | L-GSCs are able to form larger clones compared to S-GSCs, after a culture in limit dilution | | | | | |
| *Freeze*/*thaw* | Freezing of GSCs is possible by using: 50% PL+GH medium, 40% PL, 10% DMSO, with preservation of their proliferation and multipotency properties | | | | | |
| *Safety test* | No significant DNA lesions of L-GSCs were found when compared to S-GSCs and UV-irradiated gingival cells (Ctrl+) | | | | | |
| *Genotype test* | L-GSCs express similar phenotypic markers. | | | | | |
| *Differentiation test* | L-GSCs are able to maintain their potential for differentiation. | | | | | |
| | **Their osteoblastic differentiation potential is very much improved** compared to S-GSC | | | | | |
| **Osteoblastic differentiation in serum-free medium (PL medium)** | 2D et 3D | DMEM-LG GlutaMAX® and pyruvate supplement (Gibco-Life Technologies, Carlsbad, CA, USA) | 5% | L-Ascorbic acid 2-phosphate (50µg/ml; Sigma-Aldrich) | 2x per week | 21 to 28 days |
| | | | | 100nM dexamethasone | | |
| | | | | 10mM β-glycerophosphate | | |
| | | | | 10ml/l Penicillin-Streptomycin (5UI/ml, Gibco) | | |
| | | | | 1% non-essential aminoacids (NEAA; Gibco) | | |
| | | | | 2,5mg/l d'amphotericin B (250µg/ml ; Gibco) | | |
| | | | | Heparin 0,6UI/ml | | |
| *2D culture* | The osteogenic medium PL 5% allows to obtain more mineral nodules than the medium supplemented with FSC | | | | | |
| *3D culture* | Culture on allogeneic bone particles for 21 days shows that the cells spread and form pseudopods with mineral nodules in both media with PL and FCS | | | | | |

| | | | | | | |
|---|---|---|---|---|---|---|
| **DMEM-LG** : Dulbecco's Modified Eagle Medium Low Glucose **FCS :** Foetal Calf Serum **GH** : Growth Hormone **PL** : Platelet Lysate | | | | | | |

### II- Detailed results

### Proliferation test

It was first demonstrated that GSCs amplified in a 10% PL medium showed a significantly higher proliferation rate than 1%, 2.5% and 5% PL media (p<0.05%). However, this rate remained lower than that obtained in a traditional FCS 10% medium.

The Inventors also looked for elements in the FCS components that the PL did not contain and that could impact cell proliferation. Growth hormone (GH) and testosterone were selected and added to the 10% PL medium. In addition, the Inventors also assumed that the concentrations of growth factors present in the PL (FGFb, EGF, etc.) were not sufficient in some PL batches to support the proliferation of GSCs in the absence of FCS. Indeed, PL batches may show variations related to donors as well as in the manufacturing method (number of freeze/thaw cycles, storage method), which could impact the quality and content of growth factors (Doucet et al., 2005; Bernardi et al., 2013) [17, 18].

The results of cellular proliferation in serum-free medium in the presence of GH with 10% PL medium showed significantly higher proliferation rates than the 10% PL medium alone. This was also confirmed by 24-hour flow cytometry (CFSE) results where an increase in phase S was noted in this new PL 10% + GH medium.

### Primary culture and CFU-F

In addition, the isolation of GSCs (phase 1) in the serum-free medium PL 10% + GH was as effective as the isolation of GSCs in the classical 10% FCS medium, by the explant method according to the well-known method to the skilled person, as shown in Figure 1. However a denser cell population was observed at 3 weeks in L-GSC than in S-GSC. The population was named L-GSC when obtained with the serum-free medium PL 10% + GH, in contrast to the population named S-GSC when obtained with the classical 10% FCS medium.

This was the first time that a culture medium for GSC proliferation has been supplemented with GH for the growth and isolation of these cells.

The PL10% + GH medium has preserved the properties of L-GSCs and improved the ability to form clones.

### Freeze/thaw

Cryopreservation of GSCs was possible in 50% PL medium 10% + GH, 40% PL10%, 10% DMSO, at -80°C, for up to 3 months. Beyond that, the cells presented more difficulty to recover.

Figure 2 showed the freeze/thaw of L-GSCs in the PL medium with preservation of their multipotency. Indeed Figure 2B showed the ability of L-GSCs to differentiate into osteoblasts, and figure 2C showed another ability of L-GSCs to differentiate into adipocytes.

### Safety test

Figure 3 showed the absence of significant DNA damage in L-GSC cells (figure 3C) compared to S-GSC cells (figure 3B) and UV-irradiated gingival fibroblasts (Ctrl+) (figure 3A), by Hoechst staining (Anti H2ax) according to a protocol well-known to the skilled person.

### Phenotype test

If the morphology of isolated L-GSCs differs from that of S-GSGs, as already reported in the literature for MSCs (Vogel et al., 2006; Chevallier et al., 2010) [19, 20], L-GSCs express the same intra-cellular and extra-cellular phenotypic markers as S-GSCs.

### Differentiation test

The maintenance of the differentiation capacity of L-GSCs clearly showed the positive effect of this production and isolation method, which has enabled the multipotency properties to be maintained with a very significant increase in osteogenic and adipogenic differentiation.

Figure 4 showed the differentiation into osteoblasts (phase 2) by an inductive medium containing either FSC or PL, of stem cells (GSC) or control cells (GF with less than 5% stem cells) previously obtained (phase 1) in a culture medium with platelet lysate (L-GSF and L-GSC) or a culture medium with FSC (S-GSC and S-GF).

Figure 4 showed two phenomena: first, GSCs produced more mineralized tissue than GFs, secondly and more importantly, PL was much more effective that FCS at inducing mineralization. Indeed the highest mineralization rate was observed when phases 1 and 2 were carried out in PL medium (see last histogram in figure 4). Thus it was shown an improvement in osteogenic potential and mineral nodule formation for L-GSCs and in a serum-free osteogenic medium supplemented with PL.

Without being limited by this explanation, osteogenic differentiation could be explained by the effect of TGFβ1 in the early stages of differentiation (Zhao et al., 2009) [21] as well as other platelet growth factors, through mechanisms that remain to be explored. GH could also have a role in increasing the osteoblastic differentiation potential of GSCs, either by increasing their multipotency capacity by increasing the TGFβ1 rate, or by acting directly on osteoblastic differentiation.

The development of an osteogenic medium supplemented with PL for the differentiation of GSCs has been very difficult. The concentrations of PL5% and 10% were found to be the most suitable for GSCs. Heparin concentration was also important for the culture of GSCs in media containing PL. Regarding osteoblastic differentiation, this concentration of heparin was significant. Indeed, a concentration at 2 IU/ml, as recommended by the manufacturer, prevented osteoblastic differentiation in early experiments or significantly reduced its potential in some strains of GSC. The lowest possible heparin concentration (0.6 IU/ml) that is effective in preventing gel formation (coagulation) was therefore used. Cultured on a 3D matrix of porous allogenic bone (allograft Zimmer) showed good viability (Calceïn AM) and the presence of GSC mineralization around and between the nodules of this matrix. Figure 5 showed the formation of mineral pseudopods and nodules for S-GSC and L-GSC. However it must be noted that the matrix is much better organized in the PL osteogenic medium (serum-free) than in the FSC osteogenic medium. It thus confirmed the possibility of transfer of L-GSC cells in humans for bone reconstruction. This makes it possible to consider the transfer of L-GSC cells in humans for bone reconstruction.

The adipocyte differentiation of L-GSCs was significantly increased under serum-free adipogenic conditions compared to S-GSCs. Finally, the ability to differentiate to the myofibroblastic pathway and the formation of neurospheres could have therapeutic applications in wound healing and nerve damage in humans with these newly isolated cells.

The study of the immune profile of peripheral blood stem cells (PBMCs) cocultured with GSCs in the presence of antigenic particles showed that in these conditions, cytotoxic memory and regulatory T cells are significantly increased by the presence of L-GSCs. This showed the preservation of the role of GSCs in maintaining immune balance in lymphocyte populations during an inflammatory reaction. CD4+ CD3+ CD3+ CD25+ CD25+ FoxP3+ T cell proliferation was increased in the presence of S-GSC and L-GSC, as well as in the presence of PL10%+GH medium alone. This showed that S-GSC and L-GSC had the same immunomodulatory properties and that the new medium acted on the lymphocyte profile by increasing the CD4+ regulatory population. Also, from a clinical point of view, the immunomodulatory properties of GSCs could be used to improve bone grafting, especially allogeneic.

In conclusion, GSCs in the serum-free medium supplemented with PL (preferably PL10%) and GH allowed these cells to retain their cellular phenotypes and properties required for MSCs, with an improved ability to form clones and differentiate towards the osteoblastic pathway. The immunomodulatory properties of GSCs have also been preserved with a likely role of PL alone on immunomodulation by reducing CD8+ CD25+ and CD8+ CD45RO+ populations and inducing CD4+ CD3+ CD25+ FoxP3+ population.

### List of references

1. Listl et al., Journal of Dental Research, 0022034515602879, 2015
2. Petersen et Yamamoto, Community Dentistry and Oral Epidemiology, 33(2) : 81-92, 2005
3. Gundlach et Christina, Journal of Cranio-Maxillofacial Surgery, 34 : 1-2, 2006
4. Montero et Patel, Surgical Oncology Clinics of North America, 24(3) : 491, 2015
5. Leucht et al., Development, 135(17) : 2845-2854, 2008
6. Jacques et al., Connective Tissue Research, 55(sup1) : 117-120, 2014
7. Nassif, These « Role des cellules orales dérivées des crêtes neurales dans la morphogenèse craniofaciale », 2016
8. Najar et al., Cytotherapy, 18(2) : 160-171, 2016
9. Seifert et al., World Journal of Stem Cells, 7(3) : 583, 2015
10. d'Aquino et al., Eur. Cell Mater., 18(7) : 75-83, 2009
11. Fournier et al., Tissue Engineering Part A, 16(9) : 2891-2899, 2010
12. Zhang et al., The Journal of Immunology, 183(12) : 7787-7798, 2009
13. Chen et al., ARthritis & Rheumatism, 65(5) : 1181-1193, 2013
14. Tomar et al., Biochemical and Biophysical Research Communications, 393(3) : 377-383, 2010
15. Wang et al., Stem Cells and Development, 20(12) : 2093-2102, 2011
16. Naveau et al., Journal of Periodontology, 82(4) : 632-641, 2011
17. Doucet et al., Journal of Cellular Physiology, 205(2) : 228-236, 2005
18. Bernardi et al., Cytotherapy, 5(8) : 920-929, 2013
19. Vogel et al., Platelets, 17(7) : 462-469, 2006
20. Chevallier et al., Biomaterials, 31(2) : 270-278, 2010
21. Zhao et al., Tissue Engineering part A, 16(2) : 725-733, 2009

## Claims

1. A method for inducing differentiation of neuroectodemic oral stem cells into osteoblasts, said method comprising:
(a) a step of cell proliferation of neuroectodermal oral stem cells in a serum-free base culture medium supplemented with platelet lysate (PL) and human growth hormone (GH);
b) an osteoblastic differentiation step of the cell culture resulting from step a) in a serum-free base culture medium supplemented with a platelet lysate (PL) and at least one osteoblastic differentiation factor.

2. Method according to claim 1, wherein the culture of step a) is carried out up to a confluence of 80%.

3. Method according to claim 1 or 2, wherein the culture of step b) is carried out during 21 to 28 days.

4. Method according to any of claim 1 to 3, wherein the culture medium of steps a) and b) is further supplemented with heparin.

5. Method according to claim 4, wherein the culture medium of step a) contains from 1-2 IU/ml of heparin, and/or the culture medium of step b) contains from 0.1-0.6 IU/ml of heparin.

6. Method according to any of claims 1 to 5, wherein said at least one osteoblastic differentiation factor is chosen from the group consisting of corticosteroids and glycerol phosphoric esters.

7. Method according to claim 6, wherein the said at least one differentiation factor is dexamethasone and/or β-glycerophosphate.

8. Method according to any of claims 1 to 7, wherein the neuroectodermic oral stem cells are derived from gingival tissue.

9. Cell population comprising osteoblasts produced by the method according to any of claims 1 to 8, said osteoblasts expressing the homeogen Msx2.

10. Cell population as defined in claim 9, for use in cell therapy.

11. Cell population for use according to claim 10, for the restoration of bone defects of maxillary bones.

12. Culture medium for the proliferation of oral neuroectodermal stem cells comprising a serum-free base culture medium supplemented with platelet lysate (PL) and human growth hormone (GH), and optionally heparin at a concentration ranging from 1-2 IU/ml.

13. Culture medium for the differentiation of neuroectodermal oral stem cells into osteoblasts comprising a serum-free base culture medium supplemented with a platelet lysate (PL) and at least one osteoblastic differentiation factor, and optionally heparin at a concentration ranging from 0.1-0.6 IU/ml.

14. Culture medium according to claim 13, wherein said at least one osteoblastic differentiation factor is chosen from the group consisting of corticosteroides and glycerol phosphoric esters.

15. Culture medium according to claim 14, wherein said at least one osteoblastic differentiation factor is dexamethasone and/or β-glycerophosphate.
